(19) Europäisches Patentamt
European Patent Office
Office européen des brevets

(11) Veröffentlichungsnummer: **0 268 916 B1**

(12) # EUROPÄISCHE PATENTSCHRIFT

(45) Veröffentlichungstag der Patentschrift: **25.03.92**

(21) Anmeldenummer: **87116509.8**

(22) Anmeldetag: **09.11.87**

(51) Int. Cl.5: **C07C 69/34**, C07C 67/08, C07C 69/75, C07C 69/80, C07C 69/753, C07C 69/40, C07C 69/60, C07C 69/42, C08F 2/28, B01F 17/00

(54) **Bifunktionelle Emulgatoren auf Basis von Perhydrobisphenolen und Carbonsäureanhydriden.**

(30) Priorität: **22.11.86 DE 3639904**

(43) Veröffentlichungstag der Anmeldung:
**01.06.88 Patentblatt 88/22**

(45) Bekanntmachung des Hinweises auf die Patenterteilung:
**25.03.92 Patentblatt 92/13**

(84) Benannte Vertragsstaaten:
**DE ES FR GB IT NL**

(56) Entgegenhaltungen:
**US-A- 4 128 437**

(73) Patentinhaber: **BAYER AG**

**W-5090 Leverkusen 1 Bayerwerk(DE)**

(72) Erfinder: **Schmidt, Adolf, Dr.**
**Roggendorfstrasse 67**
**W-5000 Köln 80(DE)**
Erfinder: **Eichenauer, Herbert, Dr.**
**Goethestrasse 71**
**W-4047 Dormagen(DE)**
Erfinder: **Pischtschan, Alfred, Dr.**
**Zur Eiche 33**
**W-5067 Kürten(DE)**

**EP 0 268 916 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von Reaktionsprodukte, aus 1 Mol 2,2-Bis-(4-hydroxycyclohexyl)-propanen der Formel I

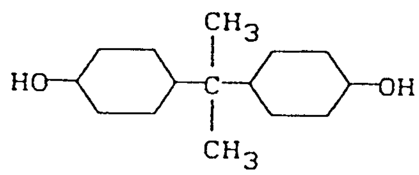

I

mit 2 Mol Carbonsäureanhydriden der Formel II

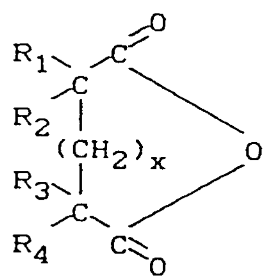

II

wobei bei x = 0
$R_1$, $R_2$, $R_3$ und $R_4$ Wasserstoff
oder
$R_1$ und/oder $R_3$: $C_1$ bis $C_{35}$-Alkyl oder Alkenyl und $R_2$ und $R_4$ sowie gegebenenfalls $R_1$ oder $R_3$ Wasserstoff
oder
$R_1$ und $R_4$ zusammen eine chemische Bindung und $R_2$ und $R_3$ Wasserstoff
oder
$R_1$ und $R_4$ zusammen eine chemische Bindung und $R_2$ und $R_3$ zusammen mit den Kohlenstoffatomen eine o-Phenylengruppe
oder
$R_1$ und $R_4$ zusammen mit den beiden CH-Gruppen (bei $R_2$ und $R_3$ gleich Wasserstoff) einen zweiwertigen Cyclohexen, Cyclohexan, Norbornen, Norbornanring,
bei x = 1
$R_1$, $R_2$, $R_3$, $R_4$, Wasserstoff
bedeuten,
die durch Zusammenschmelzen der Komponenten bei Temperaturen von 100 bis 300 °C unter Inertgas erhältlich sind, in Form ihrer Alkali- oder Ammoniumsalze als Emulgatoren zur Emulsionspolymerisation

Diese Reaktionsprodukte werden im folgenden als Emulgatorharze bezeichnet.

Aus der US-Patentschrift 4 128 437 sind unter anderem Ester von 2,2-Bis-(4-hydroxycyclohexyl)-propan mit Dicarbonsäuren oder Dicarbonsäureanhydriden wie Phthalsäureanhydrid oder Hexahydrophthalsäureanhydrid bekannt. Sie werden dort verwendet, um Papier transparent zu machen.

Verbindungen der Formel I entstehen bei der Hydrierung von Bisphenol A. Hierbei können Gemische aus dem trans-trans, dem cis-trans sowie dem cis-cis-Isomeren entstehen (vgl. Us-PS 4 487 979). Solche Produkte sind handelsüblich, z.B. "Perhydrobisphenol" der BAYER AG Leverkusen, mit einer Reinheit (gaschromatographisch) von mindestens 90 %.

Als Carbonsäureanhydride der Formel II zur Herstellung der Emulgatorharze sind z.B. geeignet: Bernsteinsäureanhydrid (III), Alkylbernsteinsäureanhydride mit 1 bis 35 C-Atomen im Alkylrest oder Alkylenrest (IV) und (V), Maleinsäureanhydrid (VI), Phthalsäureanhydrid (VII), Tetrahydrophthalsäureanhydrid (VIII), Hexahydrophthalsäureanhydrid (IX), 5-Norbornen-2,3-dicarbonsäureanhydrid (X), Norbornancarbonsäureanhydrid (XI), Glutarsäureanhydrid (XII). Die Anhydride sind nachfolgend formelmäßig angeführt.

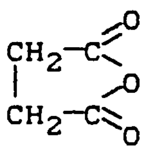 ; 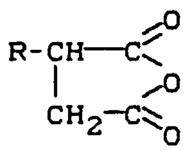 ; 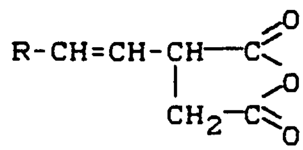 ;

(III) (IV) R = Alkyl (V) R = Alkyl

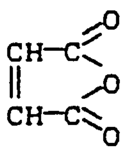 ; 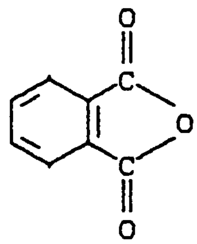 ; 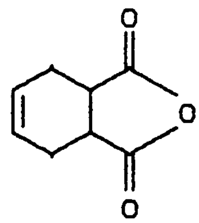 ; 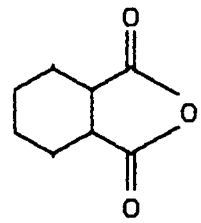

(VI) (VII) (VIII) (IX)

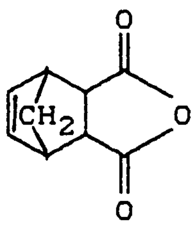 ; 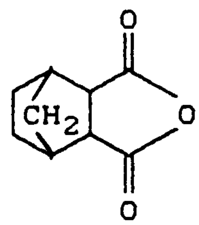 ; 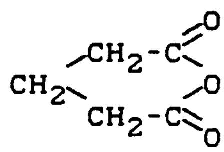

(X) (XI) (XII)

Bevorzugte Anhydride ergeben nach der Reaktion mit dem Perhydrobisphenol saure, helle Harze mit Erweichungspunkten oberhalb 50 ° C und lassen sich pulverisieren.

Bevorzugte Emulgator-Harze werden bei der Umsetzung von Perhydrobisphenol mit Phthalsäureanhydrid, Tetrahydrophthalsäureanhydrid, Hexahydrophthalsäureanhydrid, 5-Norbornen-2,3-dicarbonsäureanhydrid, Norbornandicarbonsäureanhydrid (im Molverhältnis 1:2) erhalten.

Die bevorzugten Anhydride können auch in Kombination mit den Anhydriden (III) bis (VI), vorzugsweise (VI), eingesetzt werden, indem man mit 1 Mol Perhydrobisphenol 1 Mol Maleinsäureanhydrid und 1 Mol eines Anhydrids (III) bis (V) oder (VII) bis (XII) umsetzt.

Werden 2 Mol Anhydrid der Formel (IV) oder (V) mit 1 Mol Perhydrobisphenol umgesetzt, so sind die Reaktionsprodukte nur noch in wäßrigem Alkali leicht auflösbar, wenn der Rest R nicht mehr als 18 C-Atome besitzt. Wird ein Anhydrid der Formel (IV) oder (V) mit sehr langem Rest R (z.B. R = $C_{33}$-Alkyl in Formel V) verwendet, so ist es zweckmäßig, es mit Maleinsäureanhydrid, Bernsteinsäureanhydrid oder Glutarsäureanhydrid im Molverhältnis 1:1 zu kombinieren.

Die Umsetzung in der Schmelze kann mit Hilfe von Katalysatoren, beispielsweise sehr geringen Mengen eines tertiären Amins, wie Triethylamin, Tributylamin, 1,4-Diazabicyclooctan, beschleunigt werden. Es ist empfehlenswert, die exotherme Reaktion bei starker Durchmischung durchzuführen. Ihr Ende ist durch Bestimmung der Säurezahl der Schmelze und durch Verschwinden der Anhydridbande im Infrarotspektrum erkennbar. Als Inertgas eignet sich bei der Reaktion Stickstoff oder Argon.

Zur erfindungsgemäßen Verwendung als Emulgatoren werden die zunächst sauren und kaum wasserlöslichen Reaktionsprodukte mit Alkalilaugen, Ammoniak oder Aminen neutralisiert. Bevorzugt ist wäßrige Kalilauge, da sich die Kaliumsalze der Reaktionsprodukte aufgrund ihrer guten Wasserlöslichkeit besonders als Tenside und Emulgatoren eignen.

Die Neutralisation der Reaktionsprodukte mit wäßriger Alkalilauge kann konduktometrisch verfolgt werden. Hierzu löst man eine genau bekannte Menge (ca. 2 bis 3 g) des Reaktionsproduktes in 100 ml reinem Isopropanol bei Zimmertemperatur auf und titriert konduktometrisch mit 1 n Kalilauge.

Die neutralisierten, alkalisch reagierenden Emulgatorharzlösungen können vorteilhaft als Emulgatoren bei der Emulsionspolymerisation radikalisch polymerisierbarer ethylenisch ungesättigter Monomeren und von Dienen wie Butadien, Isopren, Chloropren oder zu deren Mischpolymerisation verwendet werden.

Es besteht allgemein ein Bedarf an thermostabilen, schwer flüchtigen, gut mit dem Polymerisat

verträglichen Emulgatoren, welche zu problemlos koagulierbaren selbst aber koagulatfrei herstellbaren Dispersionen führen. Die Dispersionen sollen bei der Entfernung der Restmonomeren nicht schäumen.

Die erfindungsgemäßen Emulgatorharze werden diesen Anforderungen weitestgehend gerecht.

Durch Zusätze von Mineralsäuren, organischen Säuren, Magnesium- oder Calciumsalzen oder Kochsalz lassen sich die mit Hilfe der neuartigen Emulgatoren hergestellten Dispersionen leicht koagulieren, wobei die schwer wasserlöslichen Emulgatorsäuren oder deren schwerlösliche Ca- oder Mg-Salze im Polymerisat verbleiben. Die Koagulate lassen sich gut auswaschen und die bei üblichen Emulgatoren zu beobachtende Trübung und das Schäumen der Waschwässer solcher Koagulate unterbleibt.

Die gute Emulgatorwirkung und die Tensideigenschaften der erfindungsgemäßen harzartigen Reaktionsprodukte war nicht vorhersehbar. Sie entsprechen nicht der herkömmlichen Vorstellung von einem Emulgator als einem Molekül mit einem hydrophilen Teil und einer daran befindlichen langkettigen hydrophoben Gruppe (z.B. $C_{10}$- bis $C_{20}$-Alkyl).

So ist beispielsweise dem Reaktionsprodukt von Perhydrobisphenol und Hexahydrophthalsäureanhydrid bzw. dessen Alkalisalz (XIII) der Tensidcharakter keineswegs anzusehen:

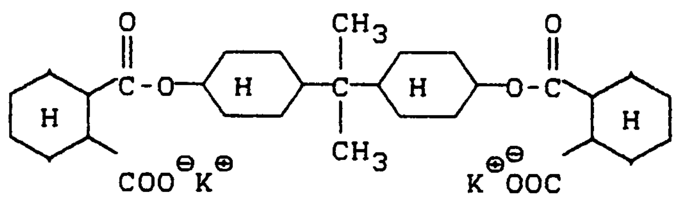

(XIII)

Die Struktur der in Formel (XIII) dargestellten Dicarbonsäure(n) ergab sich aus der Säurezahl sowie einer Molekulargewichtsbestimmung nach der Methode FAB (Fast Atom Bombardement), Negativmessung in Triethanolamin. Der signifikante Peak ist M/Z = 547 $(M\text{-}H)^{\ominus}$ (Literatur: M.Barber, R.S. Bordoli, R.D. Sedwik und A.N. Tyler, J. Chem. Soc. Chem. Commun. 325 (1981)).

Es ist nicht zu erwarten, daß Verbindungen wie das Dikaliumsalz XIII gute Mizellbildner sind, welche die Emulsionspolymerisation im Sinne der allgemein entwickelten Vorstellungen (HARKINS) ermöglichen. Die nachfolgend beispielhaft beschriebenen Polymerisationen von Monomeren mit relativ geringer Wasserlöslichkeit (Styrol und Butadien) zeigen dagegen die überraschend gute Wirksamkeit der erfindungsgemäßen Emulgatorharze.

Zur Demonstration der Abhängigkeit der Teilchengröße von Emulgatormenge wurde beim Styrol eine umfangreiche Polymerisationsserie durchgeführt (vgl. nachfolgende Beispiele 13-85).

Neben den genannten Monomeren Styrol und Butadien lassen sich die erfindungsgemäßen Emulgatoren auch zur Polymerisation von Acrylaten, Methacrylaten, zur Copolymerisation von Acrylnitril und/oder Methacrylnitril mit Styrol, alpha-Methylstyrol, Acrylaten und Methacrylaten einsetzen. Genannt sei insbesondere die Copolymerisation Butadien-Acrylnitril.

Die erfindungsgemäßen Emulgatoren lassen sich auch mit anderen an sich bekannten anionischen oder nichtionogenen Tensiden kombinieren. Die Polymerisationen können diskontinuierlich, kontinuierlich oder mit Monomerenzulauf durchgeführt werden.

Einige der erfindungsgemäßen Harze, z.B. die Umsetzungsprodukte mit Maleinsäureanhydrid oder Bernsteinsäureanhydrid mit Erweichungspunkten unter 40 ° C, zeigen eine Klebrigkeit, ähnlich wie natürliche Harze. Die Klebrigkeit kann bei der Verarbeitung beispielsweise von Butadien-Styrol-Polymerisaten durchaus erwünscht sein.

Die erfindungsgemäßen Emulgator-Harze weisen gegenüber den natürlichen Harzen einen besseren Farbton und konstante emulgierende Eigenschaften auf.

Zur Entfernung restlicher Mengen an Ausgangsmaterialien z.B. an Perhydrobisphenol, insbesondere von Anhydriden oder Wasser können die Emulgatorharze nach beendeter Reaktion im Hochvakuum erhitzt und flüchtige Anteile abdestilliert bzw. absublimiert werden.

Beispiele 1-12

Herstellung der Emulgatorharze

In Tabelle I wird die Herstellung einer Reihe (1-12) saurer Emulgatorharze auf Basis Perhydrobisphenol

(Handelsprodukt der BAYER AG) und verschiedener Carbonsäureanhydride (vgl. Spalte 2) beschrieben.

Die Komponenten wurden in den angeführten Mengen bei der in Spalte 4 genannten Temperatur während der in Spalte 5 genannten Dauer unter Rühren in der Schmelze erhitzt, danach noch flüssig abgelassen und gegebenenfalls pulverisiert. In Spalte 7 der nach konduktometrischer Titration ermittelte Verbrauch in ml 1n KOH pro Gramm saurem Harz angeführt, woraus sich die Säurezahl berechnet (Spalte 8, SZ 1).

Schließlich ist in Spalte 9 die berechnete Säurezahl eines Reaktionsproduktes angegeben, bei welchem sich 1 Mol Perhydrobisphenol mit 2 Mol Anhydrid zur alpha-omega-Dicarbonsäure umgesetzt hätte. Die beiden unterschiedlichen Säurezahlen sind mit SZ-1 und SZ-2 bezeichnet. In den meisten Fällen stimmen die beiden Säurezahlen recht gut überein.

Beispiele 13-84

Polymerisation des Styrols mit Hilfe der Emulgatorharze

Die gemäß Beispielen 1-12 erhaltenen sauren Emulgatorharze werden in der gemäß ermittelten Säurezahl SZ-1 erforderlichen wäßrigen KOH gelöst und auf einen Feststoffgehalt von 5 Gew.-% gebracht. In Tabelle IIa ist nun eine ansteigende Emulgatormenge (Zeile 3a bis f) angeführt, mit Hilfe welcher die jeweilige Emulsionspolymerisation einer bestimmten Styrolmenge (114 g) bei 70 ° C und während 7 Stunden in geschüttelten Flaschen unter Luftausschluß durchgeführt wurde. Der Monomerenumsatz war nach dieser Zeit nahezu quantitativ.

In Tabelle IIb ist die Auswertung dieser 72 Polymerisationsversuche nach Koagulatmenge und Teilchendurchmesser vorgenommen. Der Teilchendurchmesser wurde turbidimetrisch bestimmt und die angegebenen Werte entsprechen dem Durchmesser aus dem Volumen (DAV) (vgl. DIN 53206).

Tabelle I

| [g] Perhydro-bisphenol | umgesetzt mit | | [g] | Bsp. Harz Nr. | Reaktions-temperatur [°C] | Reaktions-zeit [min] | Erwei-chungs-punkt des Harzes [°C | Verbrauch ml 1n KOH/g Substanz | SZ-1 | SZ-2 |
|---|---|---|---|---|---|---|---|---|---|---|
| 120 | 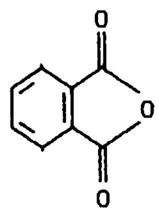 | Phthalsäure-anhydrid | 148 | 1 | 210 | 15 | 180 | 3,76 | 211 | 209 |
| 180 | 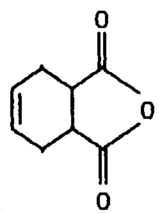 | Tetrahydro-phthalsäure-anhydrid | 228 | 2 | 130 | 180 | 88 | 3,91 | 219,4 | 206 |

<u>Tabelle I</u> (Fortsetzung)

| [g] Perhydro-bisphenol | umgesetzt mit | | [g] | Bsp. Harz Nr. | Reaktions-temperatur [°C] | Reaktions-zeit [min] | Erwei-chungs-punkt des Har-zes [°C | Verbrauch ml 1n KOH/g Substanz | SZ-1 | SZ-2 |
|---|---|---|---|---|---|---|---|---|---|---|
| 120 | 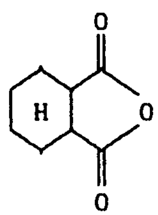 | Hexahydro-phthalsäure-anhydrid | 154 | 3 | 170 | 150 | 100 | 3,71 | 208,1 | 205 |
| 240 | 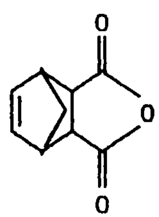 | 5-Norbornen-2,3-dicarbonsäure-anhydrid | 328,3 | 4 | 160 | 900 | 108 | 3,44 | 193,0 | 198 |

Tabelle I (Fortsetzung)

| [g] Perhydro-bisphenol | umgesetzt mit | [g] | Bsp. Harz Nr. | Reaktions-temperatur [°C] | Reaktions-zeit [min] | Erwei-chungs-punkt des Har-zes [°C | Verbrauch ml 1n KOH/g Substanz | SZ-1 | SZ-2 |
|---|---|---|---|---|---|---|---|---|---|
| 216 | 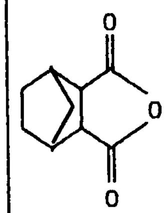 Norbornan-2,3 dicarbonsäure-anhydrid | 299 | 5 | 140 | 1.300 | 96 | 3,65 | 204,8 | 196 |
| 180 | 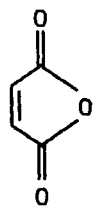 Maleinsäure-anhydrid | 147 | 6 | 125 | 240 | 35 | 4,70 | 263,7 | 257 |
| 180 | 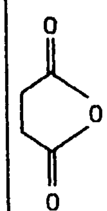 Bernstein-säureanhydrid | 150 | 7 | 145 | 240 | 35 | 4,63 | 258,1 | 255 |

Tabelle I (Fortsetzung)

| [g] Perhydro-bisphenol | umgesetzt mit | [g] | Bsp. Harz Nr. | Reaktions-temperatur [°C] | Reaktions-zeit [min] | Erwei-chungs-punkt des Har-zes [°C | Verbrauch ml 1n KOH/g Substanz | SZ-1 | SZ-2 |
|---|---|---|---|---|---|---|---|---|---|
| 180 | 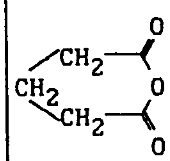 Glutarsäure-anhydrid | 171 | 8 | 130 | 240 | -8 | 4,46 | 250,2 | 240 |
| 120 | n-Octylsuccinanhydrid | 212 | 9 | 115 | 115 | -5 | 3,54 | 198,6 | 169 |
| 90 | n-Dodecylsuccinanhydrid | 243 | 10 | 115 | 115 | ±0 | 3,00 | 168,3 | 145 |
| 72 | n-Octadecylsuccinanhydrid | 211 | 11 | 140 | 240 | 28 | 1,90 | 106,6 | 119 |
| 180 | Maleinsäureanhydrid<br>Hexahydrophthalsäureanhydrid | 73,5<br>118 | 12 | 125 | 240 | 72 | 4,24 | 237,9 | 238 |

<u>Tabelle IIa</u>; Polymerisationstemperatur 70° C; Polymerisationszeit 7 [h]

| | a | b | c | d | e | f |
|---|---|---|---|---|---|---|
| Entionisiertes Wasser | 201,4 | 190,6 | 168,9 | 147,3 | 125,6 | 60,6 |
| Styrol | 114,0 | → | → | → | → | → |
| 5 %ige Emulgatorlösung (K-Salze) | 11,4 | 22,8 | 45,6 | 68,4 | 91,2 | 159,6 |
| 1 %ige $K_2S_2O_8$-Lösung | 34,2 | → | → | → | → | → |
| 1 %ige $Na_2CO_3$-Lösung | 20,1 | → | → | → | → | → |

**Tabelle IIb**

| Bsp. | Emulgator aus Harz Nr..../K-Salz | Koagulatmenge [g] bei praktisch vollständigem Umsatz des Styrols | | | | | | Teilchendurchmesser (nm) | | | | | |
|---|---|---|---|---|---|---|---|---|---|---|---|---|---|
| | | a | b | c | d | e | f | a | b | c | d | e | f |
| 13-18 | ...1 | K* | 11 | 1,3 | 0,6 | 0,4 | 0,0 | - | 125 | 100 | 105 | 112 | 120 |
| 19-24 | 2 | K | 2 | 1,1 | 0,3 | 0,0 | 0,0 | - | 200 | 140 | 120 | 110 | 90 |
| 25-30 | 3 | K | 0,8 | 0,3 | 0,3 | 0,2 | 0,0 | - | 170 | 125 | 120 | 110 | 93 |
| 31-36 | 4 | K | K | 3,4 | 1,4 | 2,4 | K | - | - | 95 | 93 | 82 | - |
| 37-42 | 5 | K | K | 2,9 | 1,1 | 0,5 | 0,0 | - | - | 83 | 77 | 78 | 125 |
| 43-48 | 6 | K | K | 1,4 | 0,5 | 0,3 | 0,2 | - | - | 98 | 93 | 92 | 90 |
| 49-54 | 7 | K | K | 0,8 | 0,4 | 0,2 | 0,1 | - | - | 120 | 103 | 99 | 92 |
| 55-60 | 8 | K | K | 0,8 | 0,7 | 0,6 | 0,5 | - | - | 90 | 82 | 75 | 70 |
| 61-66 | 9 | K | K | 0,9 | 0,3 | 0,2 | 0,0 | - | - | 90 | 85 | 77 | 73 |
| 67-72 | 10 | K | 4,3 | 4,2 | 2,5 | 1,4 | 0,0 | - | 60 | 55 | 48 | 57 | 50 |
| 73-78 | 11 | K | 8,0 | 2,6 | 2,3 | 1,3 | 0,4 | - | 91 | 85 | 78 | 69 | 59 |
| 79-84 | 12 | K | K | 0,5 | 0,3 | 0,0 | 0,0 | - | - | 123 | 110 | 103 | 90 |

*K = Ansatz ist vollständig koaguliert

Beispiele 85-89

Polymerisation des Butadiens mit Hilfe der Emulgatorharze

In einen 6 Liter Edelstahlautoklaven mit stufenlos regelbarem Blattrührer (150 Upm) und mit elektronisch regelbarer Innentemperatur werden unter Ausschluß von Luftsauerstoff eingefüllt:

| | |
|---|---|
| Entionisiertes Wasser | 2.282,00 g |
| Dikaliumsalz* der Emulgatorsäure 100 % WAS | 50,00 g |
| Tert. Dodecylmercaptan | 5,00 g |
| 2,5 %ige wäßrige Kaliumperoxodisulfatlösung | 155,00 g |
| Butadien | 1.650,00 g |

Danach wird auf 65 °C aufgeheizt und bis zum Druckabfall (bis Feststoffgehalt ca, 40 Gew.-%) auspolymerisiert.

In Tabelle III bedeuten:

* "Emulgator": das Kaliumsalz des in Tabelle I, Spalte 3 angeführten Harzes.

In Tabelle III sind Polymerisationsergebnisse bei Verwendung verschiedener Dikaliumsalze aus Emulgatorharzen gemäß Tabelle I Spalte 3 angeführt. Die Koagulatbildung ist bei den untersuchten Emulgatoren gering, der Latexteilchendurchmesser ist gegenüber dem bei üblichen Emulgatoren zu erwartenden Teilchendurchmeser deutlich erhöht. Daher sind die angeführten Polybutadienlatices auch dünnflüssig und bedürfen keiner erhöhten Elektrolytzugabe.

Polybutadienlatices auf Basis dieser Emulgatoren können in an sich bekannter Weise mit Styrol-Acrylnitril bepfropft und zu ABS mit verbessertem Oberflächenglanz und verbesserter Thermostabilität verarbeitet werden.

Tabelle III Polymerisation des Butadiens

| Emulgator* aus Harz Nr... vgl. Tab. I Sp. 3 | Bsp. | Ausfall (g) trocken | Latex Feststoffgehalt % | Teilchendurchmesser DAV, (nm) |
|---|---|---|---|---|
| 1 | 85 | 20 | ca. 40 | 80 |
| 3 | 86 | kein | ca. 40 | 100 |
| 6 | 87 | 35 | 40 | 135 |
| 9 | 88 | 2,5 | 37 | 92 |
| 12 | 89 | kein | ca. 40 | 112 |

Die genannten Dispersionen lassen sich ohne Schaumbildung und damit problemlos entmonomerisieren. Beim Entmonomerisieren tritt keine Koagulatbildung auf.

Beispiel 92

Auf den gemäß Beispiel 86 hergestellten Polybutadienlatex wird Acrylnitril-Styrol aufgepfropft:

50 Gew.-Teile Polybutadien (in Form eines Latex mit einem Feststoffgehalt von 25 Gew.-%), dessen Herstellung unter Verwendung von

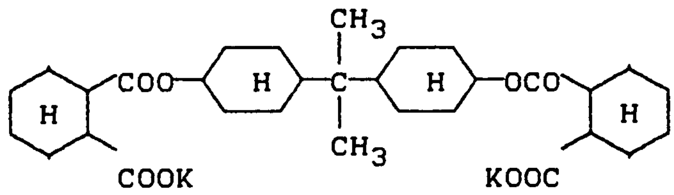

als Emulgator erfolgte, werden unter Stickstoff auf 65 ° C erwärmt, wonach 0,5 Gew.-Teile Kaliumpersulfat (gelöst in 15 Gew.-Teilen Wasser) zugegeben werden. Anschließend werden ein Gemisch aus 36 Gew.-Teilen Styrol und 14 Gew.-Teilen Acrylnitril sowie 2 Gew.-Teile des Natriumsalzes der disproportionierten Abietinsäure (gelöst in 25 Gew.-Teilen Wasser) innerhalb 4 Stunden zudosiert, wobei die Pfropfreaktion erfolgt. Nach einer Nachreaktionszeit wird der Latex in einer Magnesiumsulfat/Essigsäure-Lösung koaguliert und das resultierende Pulver bei 70 ° C im Vakuum getrocknet.

Beispiel 93

Ausprüfung einer Abmischung des unter Beispiel 92 beschriebenen Pfropfpolymerisats mit einem Styrol/Acrylnitril-Copolymerisat

40 Gew.-Teile des unter Beispiel 92 beschriebenen Pfropfpolymerisats wurden mit 60 Gew.-Teilen eines Styrol/Acrylnitril-Harzes (72:28, $M_w$ ca. 115.000; $M_w/M_n$-1 $\bar{(2)}$, 2 Gew.-Teilen Ethylendiaminbisstearylamid und 0,1 Gew.-Teilen eines Silikonöls in einem Innenkneter vermischt und anschließend durch Spritzguß zu Platten mit den Maßen 40 x 60 x 2 mm verarbeitet. Die Platten wurden hinsichtlich Rohton und Oberflächenglanz beurteilt, wobei die Glanzbeurteilung nach der Skala A-H gemäß DE-AS 2 420 358 erfolgte.

| Verarbeitungstemperatur | Rohton | Glanzstufe |
|---|---|---|
| 240 ° C | hell | F |
| 260 ° C | hell | F |
| 280 ° C | hell | E-F |

Beispiel 94

Auf den gemäß Beispiel 87 hergestellten Polybutadienlatex wird Acrylnitril-Styrol aufgepfropft

50 Gew.-Teile Polybutadien (in Form eines Latex mit einem Feststoffgehalt von 23 Gew.-%), dessen Herstellung unter Verwendung von

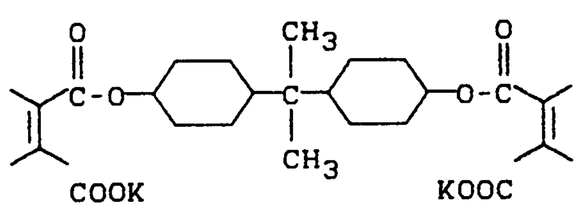

als Emulgator erfolgte, wurde in Analogie zum Beispiel 92 mit 36 Gew.-Teilen Styrol und 14 Gew.-Teilen Acrylnitril gepfropft, durch Koagulation aufgearbeitet und bei 70 ° C im Vakuum getrocknet.

Beispiel 95

Ausprüfung des unter Beispiel 94 beschriebenen Pfropfpolymerisats nach Abmischung mit einem Styrol/Acrylnitril-Copolymerisat

40 Gew.-Teile des unter Beispiel 94 beschriebenen Pfropfpolymerisats wurden mit 60 Gew.-Teilen eines in Beispiel 93 beschriebenen Styrol/Acrylnitril-Harzes sowie den dort angegebenen Additiven vermischt und durch Spritzguß verarbeitet.

Die an den Platten durchgeführten Beurteilungen ergaben folgende Werte:

| Verarbeitungstemperatur | Rohton | Glanzstufe |
|---|---|---|
| 240 °C | hell | F |
| 260 °C | hell | F |
| 280 °C | hell | E-F |

## Patentansprüche

**1.** Verwendung von Reaktionsprodukten aus 1 Mol 2,2-Bis-(4-hydroxy-cyclohexyl)-propanen der Formel I

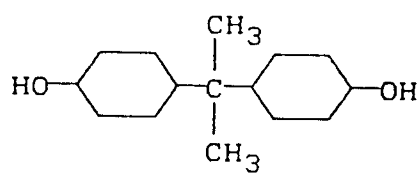

I

mit 2 Mol Carbonsäureanhydriden der Formel II

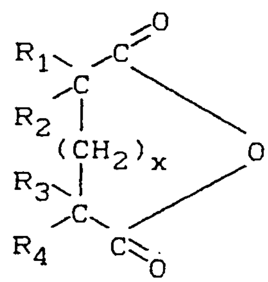

II

wobei bei x = 0

$R_1$, $R_2$, $R_3$ und $R_4$ Wasserstoff

oder

$R_1$ und/oder $R_3$ $C_1$ bis $C_{35}$-Alkyl oder Alkenyl und $R_2$ und $R_4$ sowie gegebenenfalls $R_1$ oder $R_3$ Wasserstoff

oder

$R_1$ und $R_4$ zusammen eine chemische Bindung und $R_2$ und $R_3$ Wasserstoff

oder

$R_1$ und $R_4$ eine chemische Bindung und $R_2$ und $R_3$ zusammen mit den Kohlenstoffatomen eine o-Phenylengruppe

oder

$R_1$ und $R_4$ zusammen mit den beiden CH-Gruppen (bei $R_2$ und $R_3$ gleich Wasserstoff) einen zweiwertigen Cyclohexen, Cyclohexan, Norbornen, Norbornanring,

bei x = 1

$R_1$, $R_2$, $R_3$, $R_4$ Wasserstoff

bedeuten,

erhältlich durch Zusammenschmelzen der Komponenten bei Temperaturen von 100 bis 300 °C unter Inertgas, in Form ihrer Alkali- oder Ammoniumsalze als Emulgatoren zur Emulsionspolymerisation.

## Claims

**1.** The use of reaction products of 1 mol of 2,2-bis-(4-hydroxy-cyclohexol)-propanes corresponding to formula I

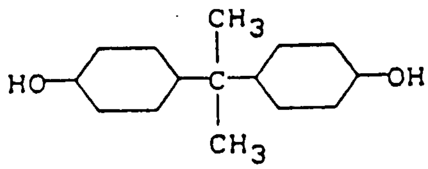

I

with 2 mol of carboxylic acid anhydrides corresponding to formula II

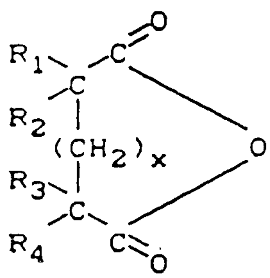

II

wherein, when x = 0,
$R_1$, $R_2$, $R_3$ and $R_4$ denote hydrogen
or
$R_1$ and/or $R_3$ denote $C_1$ to $C_{35}$-alkyl or alkenyl and
$R_2$ and $R_4$ and optionally $R_1$ or $R_3$ denote hydrogen
or
$R_1$ and $R_4$ together denote a chemical bond and $R_2$ and $R_3$ denote hydrogen
or
$R_1$ and $R_4$ denote a chemical bond and $R_2$ and $R_3$ together with the carbon atom denote an o-phenyl group
or
$R_1$ and $R_4$ together with the two CH groups (when $R_2$ and $R_3$ both stand for hydrogen) denote a divalent cyclohexene, cyclohexane, norbornene or norbornane ring,
and when x = 1,
$R_1$, $R_2$, $R_3$ and $R_4$ denote hydrogen,
obtainable by melting the components together at temperatures of from 100 to 300 °C under inert gas, in the form of their alkali metal or ammonium salts as emulsifiers for emulsion polymerisation.

## Revendications

**1.** Utilisation de produits de réaction de 1 mole de 2,2-bis(4-hydroxy-cyclohexol)-propane de formule I

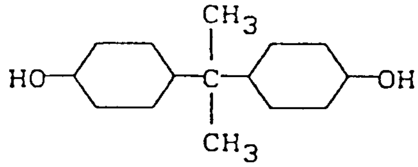

I

avec 2 moles d'anhydride d'acide carboxylique de formule II

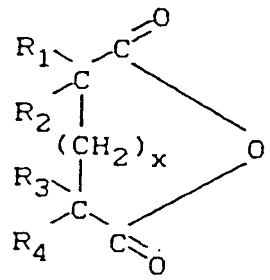

II

où x est égal à 0

$R_1$, $R_2$, $R_3$ et $R_4$ représentent l'hydrogène

ou bien

$R_1$ et/ou $R_3$ sont des groupes alkyle ou alcényle en $C_1$ à $C_{35}$ et $R_2$ et $R_4$ de même que, le cas échéant, $R_1$ ou $R_3$ représentent l'hydrogène,

ou bien

$R_1$ et $R_4$ forment conjointement une liaison chimique et $R_2$ et $R_3$ représentent l'hydrogène

ou bien

$R_1$ et $R_4$ représentent une liaison chimique et $R_2$ et $R_3$ forment conjointement avec les atomes de carbone un groupe o-phénylène

ou bien

$R_1$ et $R_4$ représentent conjointement avec les deux groupes CH (au cas où $R_2$ et $R_3$ représentent l'hydrogène) un noyau de cyclohexène, cyclohexane, norbornène ou norbornane divalent,

au cas où x est égal à 1,

$R_1$, $R_2$, $R_3$, $R_4$ représentent l'hydrogène, obtenus par fusion conjointe des composants à des températures de 100 à 300 °C sous atmosphère de gaz inerte, sous forme de leurs sels de métaux alcalins ou d'ammonium, comme émulsionnants pour la polymérisation en émulsion.